(19) 

Europäisches
Patentamt
European
Patent Office
Office européen
des brevets

(11) **EP 4 046 623 A1**

(12) **EUROPEAN PATENT APPLICATION**
published in accordance with Art. 153(4) EPC

(43) Date of publication:
**24.08.2022 Bulletin 2022/34**

(21) Application number: **20877932.2**

(22) Date of filing: **13.10.2020**

(51) International Patent Classification (IPC):
*A61K 8/891* $^{(2006.01)}$     *A61K 8/06* $^{(2006.01)}$
*A61Q 19/00* $^{(2006.01)}$

(52) Cooperative Patent Classification (CPC):
**A61K 8/06; A61K 8/37; A61K 8/891; A61Q 17/04;
A61Q 19/00**

(86) International application number:
**PCT/JP2020/038661**

(87) International publication number:
**WO 2021/075433 (22.04.2021 Gazette 2021/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.10.2019  JP 2019188722**

(71) Applicant: **Kao Corporation
Chuo-ku
Tokyo 103-8210 (JP)**

(72) Inventors:
• **FUJIOKA, Masahiro**
**Odawara-shi, Kanagawa 250-0002 (JP)**
• **TATEBE, Momoko**
**Odawara-shi, Kanagawa 250-0002 (JP)**

(74) Representative: **Hoffmann Eitle
Patent- und Rechtsanwälte PartmbB
Arabellastraße 30
81925 München (DE)**

(54) **WATER-IN-OIL EMULSION COSMETIC**

(57) A water-in-oil emulsion cosmetic comprises the following components (A), (B), (C), and (D): (A) a silicone oil (excluding component (C)); (B) a solid organic UV absorber; (C) from 0.2 to 20 mass% of a silicone oil to which an alkyl group having 8 to 28 carbon atoms is added; and (D) an ester oil, wherein a mass ratio of the component (B) to the component (C), (B)/(C), is from 0.02 to 15.

**EP 4 046 623 A1**

**Description**

Field of the Invention

The present invention relates to a water-in-oil emulsion cosmetic.

Background of the Invention

Conventionally, in order to protect the skin from the harmful effects of UV rays, skin cosmetics that provide a high UV protection effect have been demanded, and cosmetics with excellent feeling of use and stability in addition to the UV protection effect are being studied. In particular, water-in-oil emulsion cosmetics are resistant to sweat and sebum and can efficiently spread oil-soluble active ingredients on the skin and are therefore widely applied.

For example, Patent Literature 1 discloses that a water-in-oil emulsion cosmetic in which a metal oxide is mixed with propanediol dicaprylate/dicaprate, caprylyl methicone, and silicone resin-coated silicone rubber spherical powder is excellent in viscosity stability over time, feeling of use (spread), and UV-shielding effect. Patent Literature 2 discloses that a sunscreen cosmetic comprising hydrophobized zinc oxide powder, volatile silicone, and alkyl trimethicone is excellent in makeup lasting quality and cleansing easiness. Patent Literature 3 discloses that a water-in-oil emulsion cosmetic comprising polar oil, hydrocarbon oil, polyoxyalkylene alkyl co-modified organopolysiloxane, and surface-treated powder is excellent in feeling of use and powder dispersion stability.

[Patent Literature 1] JP-A-2015-189671
[Patent Literature 2] JP-A-2005-232069
[Patent Literature 3] JP-A-2018-108952

Summary of the Invention

The present invention relates to a water-in-oil emulsion cosmetic comprising the following components (A), (B), (C), and (D):

(A) a silicone oil (excluding component (C));
(B) a solid organic UV absorber;
(C) from 0.2 to 20 mass% of a silicone oil to which an alkyl group having 8 to 28 carbon atoms is added; and
(D) an ester oil, wherein

a mass ratio of the component (B) to the component (C), (B)/(C), is from 0.02 to 15.

In addition, the present invention relates to a water-in-oil emulsion cosmetic comprising the following components (A), (B), (C), (D), and (E):

(A) at least one silicone oil selected from the group consisting of a linear dimethylpolysiloxane, a branched siloxane, and a cyclic dimethylsiloxane (excluding component (C));
(B) a solid organic UV absorber;
(C) from 0.2 to 20 mass% of a silicone oil to which an alkyl group having 8 to 28 carbon atoms is added;
(D) from 1 to 20 mass% of an ester oil; and
(E) less than 5 mass% of a polyether-modified silicone surfactant, and

from 0.05 to 3 mass% of an oily gelling agent, wherein
a mass ratio of the component (B) to the component (C), (B)/(C), is from 0.02 to 15.

Detailed Description of the Invention

Conventional cosmetics are not completely satisfactory in, for example, the UV protection effect in light of the SPF standard which has become stricter these days and feeling of use.

In addition, when a water-in-oil emulsion cosmetic is prepared by adding a solid UV absorber to a conventional cosmetic, the solid organic UV absorber is hardly soluble in water and a large number of oils and is therefore difficult to be contained uniformly and stably in the cosmetic, resulting in not achieving high SPF and UV protection effect. In addition, when these components are added, there are problems, such as generation of nonuniformity and occurrence of stickiness.

The present inventors found that when a solid organic UV absorber is to be added to a cosmetic by means of an ester oil and is used in combination with a silicone oil considering feeling of use, they separate from each other, but when a silicone oil to which an alkyl group is added is further used in a specific proportion, the solid organic UV absorber can be uniformly and stably mixed in the cosmetic, and a water-in-oil emulsion cosmetic that shows a high UV protection effect, is not sticky, can be uniformly applied, and gives polished-like smooth finish can be obtained.

The water-in-oil emulsion cosmetic of the present invention has a high UV protection effect and can retain the effect for a long time. In addition, the cosmetic is not sticky, can be uniformly applied, and can finish a uniform and polished-like smooth skin, and the finish is retained for a long time. Furthermore, the skin finished to be polished-like smooth looks to have fine texture.

Incidentally, the polished-like smooth skin means that the skin looks to be three-dimensional, soft, and roundish, not flat and is in a state in which pores and micro-irregularities on the skin are not noticeable by applying the water-in-oil emulsion cosmetic, as compared to unapplied skin.

The silicone oil as the component (A) used in the present invention is an oil that is used in ordinary cosmetics excluding the component (C) and may be a volatile silicone oil or a nonvolatile silicone oil and is preferably a silicone oil that is in a liquid state at 25°C. The liquid state means to have fluidity and encompasses cream and paste.

Examples of the silicone oil as the component (A) used in the present invention include a linear dimethylpolysiloxane, a branched siloxane, a cyclic dimethyl siloxane, and modified silicone oils, such as a phenyl-modified silicone, a polyether-modified silicone, a higher fatty acid ester-modified silicone, a higher alkoxy-modified silicone, a fluorine-modified silicone, and a crosslinked organopolysiloxane. The silicone oil preferably includes one or more selected from the group consisting of a linear dimethylpolysiloxane, a branched siloxane, and a cyclic dimethyl siloxane, more preferably one or more selected from the group consisting of a linear dimethylpolysiloxane and a cyclic dimethyl siloxane, and further preferably a cyclic dimethyl siloxane.

In the volatile silicone oil, volatile means to have a flash point of 35°C to 87°C.

Examples of the volatile silicone oil include linear dimethylpolysiloxanes, such as dimethylpolysiloxane (1 cs), dimethylpolysiloxane (1.5 cs), and dimethylpolysiloxane (2 cs); branched siloxanes, such as ethyl trisiloxane, methyl trimethicone, and tetrakis(trimethylsilyl)silane; and cyclic dimethyl siloxanes, such as octamethylcyclotetrasiolxane, decamethylcyclopentasiloxane, and dodecamethylcyclohexasiloxane.

Among these examples, decamethylcyclopentasiloxane, dimethylpolysiloxane (1 cs), dimethylpolysiloxane (1.5 cs), dimethylpolysiloxane (2 cs), and methyl trimethicone are preferable.

Examples of the nonvolatile silicone oil include a dimethylpolysiloxane; and modified silicone oils, such as a higher fatty acid ester-modified silicone and a higher alkoxy-modified silicone.

Among these examples, a dimethylpolysiloxane is preferable.

Examples of the dimethylpolysiloxane include linear dimethylpolysiloxanes, such as dimethylpolysiloxane (6 cs), dimethylpolysiloxane (10 cs), and dimethylpolysiloxane (20 cs), and preferred are dimethylpolysiloxane (6 cs) and dimethylpolysiloxane (10 cs), and more preferred is dimethylpolysiloxane (6 cs).

As the component (A), from the viewpoints of light feeling of use and makeup lasting quality, volatile silicone is preferable.

In addition, from the viewpoints of a high UV protection effect, retaining the effect for a long time, and finishing the skin polished-like smooth after application, the component (A) preferably includes a volatile silicone oil, more preferably one or more selected from the group consisting of decamethylcyclopentasiloxane, dimethylpolysiloxane (1 cs), dimethylpolysiloxane (1.5 cs), dimethylpolysiloxane (2 cs), and methyl trimethicone, and further preferably one or more selected from the group consisting of decamethylcyclopentasiloxane, dimethylpolysiloxane (2 cs), and methyl trimethicone. In addition, it is even more preferable to at least include decamethylcyclopentasiloxane.

As the component (A), one or more silicone oils can be used, and the content thereof in the entire composition is preferably 10 mass% or more, more preferably 15 mass% or more, and further preferably 20 mass% or more and is preferably 55 mass% or less, more preferably 50 mass% or less, and further preferably 45 mass% or less, from the viewpoints of no stickiness and providing water repellency to the skin after application of the cosmetic. In addition, the content of the component (A) in the entire composition is preferably from 10 to 55 mass%, more preferably from 15 to 50 mass%, and further preferably from 20 to 45 mass%.

In addition, as the component (A), one or more silicone oils can be used, and the content thereof in the entire composition is preferably 10 mass% or more, more preferably 15 mass% or more, further preferably 20 mass% or more, and even more preferably 27 mass% or more and is preferably 55 mass% or less, more preferably 50 mass% or less, further preferably 45 mass% or less, and even more preferably 34 mass% or less, from the viewpoints of a high UV protection effect, retaining the effect for a long time, finishing the skin polished-like smooth after application, reducing stickiness, and providing water repellency to the skin after application of the cosmetic. In addition, the content of the component (A) in the entire composition is preferably from 10 to 55 mass%, more preferably from 15 to 50 mass%, further preferably from 20 to 45 mass%, and even more preferably from 27 to 34 mass%.

The component (B) used in the present invention is a solid organic UV absorber. Here, the solid means to be solid at 25°C.

The organic UV absorber as the component (B) may be one that is used in ordinary cosmetics, and examples thereof include 4-tert-butyl-4'-methoxydibenzoylmethane, hexyl diethylaminohydroxybenzoylbenzoate, 2-ethylhexyl dimethoxybenzylidene dioxoimidazolidine propionate, methylene bis-benzotriazolyl tetramethylbutylphenol, bis-ethylhexyloxyphenol methoxyphenyl triazine, and ethylhexyl triazone. Among these examples, from the viewpoints of good compatibility with the component (A), enhancing the UV protection effect of the cosmetic, and retaining the effect, hexyl diethylaminohydroxybenzoylbenzoate, bis-ethylhexyloxyphenol methoxyphenyl triazine, and ethylhexyl triazone are preferable.

As the component (B), from the viewpoints of a high UV protection effect, retaining the effect for a long time, and finishing the skin polished-like smooth after application, it is preferable to include one or more selected from the group consisting of hexyl diethylaminohydroxybenzoylbenzoate, bis-ethylhexyloxyphenol methoxyphenyl triazine, and ethylhexyl triazone, more preferably one or more selected from the group consisting of hexyl diethylaminohydroxybenzoylbenzoate and bis-ethylhexyloxyphenol methoxyphenyl triazine. In addition, it is preferable to use a combination of hexyl diethylaminohydroxybenzoylbenzoate and bis-ethylhexyloxyphenol methoxyphenyl triazine.

As the component (B), the solid organic UV absorbers can be used singly or in combinations of two or more, and the content thereof in the entire composition is, from the viewpoints of having a sufficient UV protection effect in a wide UV wavelength range, feeling of use, and stability, preferably 0.1 mass% or more, more preferably 0.2 mass% or more, and further preferably 0.5 mass% or more and preferably 10 mass% or less, more preferably 7 mass% or less, further preferably 5 mass% or less, and even more preferably 3 mass% or less. In addition, the content of the component (B) in the entire composition is preferably from 0.1 to 10 mass%, more preferably from 0.2 to 7 mass%, further preferably from 0.5 to 5 mass%, and even more preferably from 0.5 to 3 mass%.

In addition, as the component (B), the solid organic UV absorbers can be used singly or in combinations of two or more, and the content thereof in the entire composition is, from the viewpoints of having a sufficient UV protection effect in a wide UV wavelength range, improving the feeling of use and stability, finishing the skin polished-like smooth after application, and reducing stickiness, preferably 0.1 mass% or more, more preferably 0.2 mass% or more, further preferably 0.5 mass% or more, and even more preferably 1 mass% or more and preferably 10 mass% or less, more preferably 7 mass% or less, further preferably 5 mass% or less, even more preferably 3 mass% or less, and particularly preferably 1.5 mass% or less. In addition, the content of the component (B) in the entire composition is preferably from 0.1 to 10 mass%, more preferably from 0.2 to 7 mass%, further preferably from 0.5 to 5 mass%, even more preferably from 0.5 to 3 mass%, and particularly preferably from 1 to 1.5 mass%.

The silicone oil as the component (C) used in the present invention has an alkyl group having 8 to 28 carbon atoms added. From the viewpoints of improving the compatibility with the components (A), (B), and (D), suppressing stickiness, and further improving the makeup lasting quality and the finish of the smooth and fine textured skin, the alkyl group has preferably from 4 to 30, more preferably from 8 to 28 carbon atoms.

The silicone oil as the component (C) is preferably composed of an alkyl group and silicone. In addition, the silicone oil may have a substituent other than the alkyl group having 8 to 28 carbon atoms, but preferably does not have emulsifying capacity and more preferably does not have a polyoxyalkylene group from the viewpoints of improving the compatibility with the component (B), reducing stickiness, and further improving the makeup lasting quality.

The silicone oil as the component (C) is one that is used in ordinary cosmetics, and examples thereof include caprylyl methicone, cetyl dimethicone, alkyl(C26-28) dimethicone, stearyl dimethicone, behenoxy dimethicone, and an (acrylate/stearyl acrylate/dimethicone methacrylate) copolymer.

In particular, preferred are caprylyl methicone, cetyl dimethicone, alkyl(C26-28) dimethicone, stearyl dimethicone, and behenoxy dimethicone, and more preferred is caprylyl methicone.

Although the reason why the effect of the present invention is obtained by containing such a component (C) in the water-in-oil emulsion cosmetic of the present invention is not clear, one reason therefor is inferred that the component (C) has certain degrees of compatibility with all the components (A) and (B) and the component (D) described later and that the uniformity of the oil phase is maintained loosely when the components (A) to (D) are mixed into a water-in-oil emulsion cosmetic.

As the component (C), the silicone oils can be used singly or in combinations of two or more, and the content thereof in the entire composition is, from the viewpoints of good balance in compatibility with the components (A), (B), and (D), the UV protection effect, a reduction in stickiness, uniformity after application, high makeup lasting quality, and further improving the polished-like smooth finish, 0.2 mass% or more, preferably 0.3 mass% or more, and more preferably 0.5 mass% or more and 20 mass% or less, preferably 15 mass% or less, and more preferably 10 mass% or less. In addition, the content of the component (C) in the entire component is from 0.2 to 20 mass%, preferably from 0.3 to 15 mass%, and more preferably from 0.5 to 10 mass%.

In addition, as the component (C), the silicone oils can be used singly or in combinations of two or more, and the content thereof in the entire composition is, from the viewpoints of good balance in compatibility with the components (A), (B), and (D), the UV protection effect, a reduction in stickiness, uniformity after application, high makeup lasting quality, and further improving the polished-like smooth finish, 0.2 mass% or more, preferably 0.3 mass% or more, more preferably 0.5 mass% or more, and even more preferably 1.5 mass% or more and 20 mass% or less, preferably 15 mass% or less, more preferably 10 mass% or less, and even more preferably 5 mass% or less. In addition, the content of the component (C) in the entire composition is from 0.2 to 20 mass%, preferably from 0.3 to 15 mass%, more preferably from 0.5 to 10 mass%, and even more preferably from 1.5 to 5 mass%.

In the present invention, the mass ratio of the component (B) to the component (C), (B)/(C), is 0.02 or more, preferably 0.05 or more, and more preferably 0.1 or more and is 15 or less, preferably 5 or less, more preferably 4 or less, and further preferably 3 or less, from the viewpoints of improving the balance in compatibility and further enhancing the effects of the present invention, such as the UV protection effect. In addition, the mass ratio of the component (B) to the component (C), (B)/(C), is from 0.02 to 15, preferably from 0.02 to 5, more preferably from 0.05 to 4, and further preferably from 0.1 to 3.

In addition, in the present invention, the mass ratio of the component (B) to the component (C), (B)/(C), is 0.02 or more, preferably 0.05 or more, more preferably 0.1 or more, and even more preferably 0.2 or more and is 15 or less, preferably 5 or less, more preferably 4 or less, further preferably 3 or less, and even more preferably 1 or less, from the viewpoints of improving the balance in compatibility, further enhancing the effects of the present invention, such as UV protection effect, reducing stickiness, uniformity after application, high makeup lasting quality, and further improving polished-like smooth finish. In addition, the mass ratio of the component (B) to the component (C), (B)/(C), is from 0.02 to 15, preferably from 0.02 to 5, more preferably from 0.05 to 4, further preferably from 0.1 to 3, and even more preferably from 0.2 to 1.

In the present invention, the mass ratio of the component (C) to the component (A), (C)/(A), is preferably 0.005 or more, more preferably 0.01 or more, and further preferably 0.015 or more and is preferably 0.5 or less, more preferably 0.4 or less, and further preferably 0.3 or less, from the viewpoints of improving the balance in compatibility and further enhancing the effects of the present invention, such as UV protection effect. In addition, the mass ratio of the component (C) to the component (A), (C)/(A), is preferably from 0.005 to 0.5, more preferably from 0.01 to 0.4, and further preferably from 0.015 to 0.3. In the present invention, the mass ratio of the component (C) to the component (A), (C)/(A), is preferably 0.005 or more, more preferably 0.01 or more, further preferably 0.015 or more, and even more preferably 0.045 or more and is preferably 0.5 or less, more preferably 0.4 or less, further preferably 0.3 or less, and even more preferably 0.2 or less, from the viewpoints of improving the balance in compatibility, further enhancing the effects of the present invention, such as UV protection effect, and finishing the skin polished-like smooth after application. In addition, the mass ratio of the component (C) to the component (A), (C)/(A), is preferably from 0.005 to 0.5, more preferably from 0.01 to 0.4, further preferably from 0.015 to 0.3, and even more preferably from 0.045 to 0.2.

The ester oil as the component (D) used in the present invention is not limited as long as it is used in ordinary cosmetics and is preferably an ester oil that is in a liquid state at 25°C, and examples thereof include ester oils, such as isopropyl myristate, octyldodecyl myristate, isopropyl isostearate, isononyl isononanoate, butyl stearate, oleyl oleate, isotridecyl isononanoate, isostearyl myristate, octyldodecyl ricinoleate, diglyceryl monoisostearate, ethylhexyl palmitate, cetyl ethylhexanoate, ethylhexyl methoxycinnamate, tocopherol acetate, propylene carbonate, diisostearyl malate, neopentyl glycol dicaprate, neopentyl glycol diethylhexanate, diglyceryl diisostearate, propanediol diisostearate, glyceryl monoisostearate monomyristate, glyceryl triisostearate, propanediol dicaprylate/dicaprate, glyceryl tricaprylate/tricaprate, triethylhexanoin, trimethylolpropane tri-2-ethylhexanoate, trimethylolpropane triisostearate, pentaerythrityl tetraoctanoate, pentaerythritol tetraethylhexanoate, pentaerythritol tetraisostearate, polyglyceryl-2 isostearate, polyglyceryl-2 diisostearate, polyglyceryl-2 triisostearate, polyglyceryl-2 tetraisostearate, polyglyceryl-6 octacaprylate, (isostearic acid/sebacic acid) ditrimethylolpropane oligoester, dipentaerythrityl tripolyhydroxystearate, diphytosteryl/dioctyldodecyl lauroyl glutamate, trehalose isostearate esters, dipentaerythrityl tetraisostearate, dipentaerythrityl pentaisostearate, ethylhexyl hydroxystearate, phytosterol fatty acid ester, cholesterol fatty acid ester, polyglycerol fatty acid ester, and pentaerythritol fatty acid ester; and vegetable oils, such as avocado oil, macadamia nut oil, olive oil, rapeseed oil, sesame oil, wheat germ oil, linseed oil, cotton seed oil, soybean oil, palm oil, coconut oil, castor oil, jojoba oil, sunflower oil, camellia oil, and corn oil.

Among these examples, from the viewpoints of dissolving the component (B) and improving the uniformity of the component (B) in the coating film, esters of fatty acids and primary alcohols, such as isopropyl myristate, octyldodecyl myristate, isopropyl isostearate, isononyl isononanoate, butyl stearate, oleyl oleate, isotridecyl isononanoate, isostearyl myristate, octyldodecyl ricinoleate, diglyceryl monoisostearate, ethylhexyl palmitate, cetyl ethylhexanoate, and ethylhexyl methoxycinnamate, are preferable, and from the viewpoints of further enhancing the UV protection effect in a wide UV wavelength range in addition to the above viewpoints, ethylhexyl methoxycinnamate is more preferable.

In addition, from the viewpoints of a high UV protection effect, retaining the effect for a long time, dissolving the component (B), improving the uniformity of the component (B) in the coating film, and finishing the skin polished-like smooth after application, it is preferable to include one or more selected from the group consisting of 2-ethylhexyl paramethoxycinnamate, isononyl isononanoate, neopentyl glycol dicaprate, and diisostearyl malate, more preferably one or more selected from the group consisting of 2-ethylhexyl paramethoxycinnamate and diisostearyl malate. In addition, it is preferable to at least include 2-ethylhexyl paramethoxycinnamate.

As the component (D), the ester oils can be used singly or in combinations of two or more, and the content thereof in the entire composition is, from the viewpoints of makeup lasting quality and feeling of use, preferably 1 mass% or more, more preferably 2 mass% or more, and further preferably 5 mass% or more and preferably 20 mass% or less, more preferably 18 mass% or less, and further preferably 15 mass% or less. In addition, the content of the component (D) in the entire composition is preferably from 1 to 20 mass%, more preferably from 2 to 18 mass%, and further preferably from 5 to 15 mass%.

As the component (D), the ester oils can be used singly or in combinations of two or more, and the content thereof in the entire composition is, from the viewpoints of makeup lasting quality, improving feeling of use, a high UV protection effect, retaining the effect for a long time, and finishing the skin polished-like smooth after application, preferably 1 mass% or more, more preferably 2 mass% or more, and further preferably 5 mass% or more and preferably 20 mass% or less, more preferably 18 mass% or less, further preferably 15 mass% or less, and even more preferably 8 mass% or less. In addition, the content of the component (D) in the entire composition is preferably from 1 to 20 mass%, more preferably from 2 to 18 mass%, further preferably from 5 to 15 mass%, and even more preferably from 5 to 8 mass%.

In the present invention, the mass ratio of the component (D) to the component (C), (D)/(C), is preferably 0.5 or more, more preferably 0.7 or more, and further preferably 1 or more and is preferably 50 or less, more preferably 30 or less, and further preferably 20 or less, from the viewpoints of improving the balance in compatibility of the components (A) to (D) and further enhancing the effects of the present invention, such as UV protection effect. In addition, the mass ratio of the component (D) to the component (C), (D)/(C), is preferably from 0.5 to 50, more preferably from 0.7 to 30, and further preferably from 1 to 20.

In the present invention, the mass ratio of the component (D) to the component (C), (D)/(C), is preferably 0.5 or more, more preferably 0.7 or more, and further preferably 1 or more and is preferably 50 or less, more preferably 30 or less, further preferably 20 or less, and even more preferably 5 or less, from the viewpoints of improving the balance in compatibility of components (A) to (D), further enhancing the effects of the present invention, such as UV protection effect, and finishing the skin polished-like smooth after application. In addition, the mass ratio of the component (D) to the component (C), (D)/(C), is preferably from 0.5 to 50, more preferably from 0.7 to 30, further preferably from 1 to 20, and even more preferably from 1 to 5.

In the water-in-oil emulsion cosmetic of the present invention, from the viewpoints of not having stickiness and further improving the makeup lasting quality, the content of the silicone-type surfactant (E) is preferably less than 5 mass%, more preferably 4.5 mass% or less, further preferably 4.0 mass% or less, even more preferably 2 mass% or less, and further preferably 0.5 mass% or less. In addition, from the viewpoints of not having stickiness, more improving the makeup lasting quality, and improving the storage stability, the content is preferably 0.1 mass% or more, more preferably 0.2 mass% or more, and further preferably 0.3 mass% or more.

The silicone-type surfactant is a dimethylpolysiloxane having a polyoxyethylene group or a polyoxypropylene group or a dimethylpolysiloxane to which polyethylene glycol is added, and examples thereof include those that are used in ordinary cosmetics. Incidentally, the dimethylpolysiloxane may be partially fluorine-modified.

The silicone-type surfactant is, from the viewpoints of a high UV protection effect, retaining the effect for a long time, and finishing the skin polished-like smooth after application, preferably a polyether-modified silicone surfactant, more preferably a dimethylpolysiloxane having a polyoxyethylene group or a polyoxypropylene group, and further preferably a dimethylpolysiloxane at least having a polyoxyethylene group.

Incidentally, the silicone-type surfactant preferably does not include a crosslinked type.

Such a silicone-type surfactant preferably has an HLB of 12 or less.

In addition, from the viewpoints of a high UV protection effect, retaining the effect for a long time, and finishing the skin polished-like smooth after application, the HLB is preferably 1 or more and more preferably 3 or more and is preferably 12 or less, more preferably 10 or less, further preferably 8 or less, and even more preferably 6 or less.

Here, HLB (hydrophilic-lipophilic balance) indicates the molecular weight of the hydrophilic group portion of the total molecular weight of a surfactant and is determined by the Griffin's equation. When a surfactant is composed of two or more silicone-type surfactants, the HLB of a mixed surfactant is determined as follows. The HLB of a mixed surfactant is the arithmetic mean of the HLB values of each silicone-type surfactant based on the blending ratio.

$$\text{Mixed HLB} = \Sigma(\text{HLBx} \times \text{Wx})/\Sigma\text{Wx}$$

HLBx indicates the HLB value of a silicone-type surfactant X.

Wx indicates the mass (g) of the silicone-type surfactant X having a value of HLBx.

The water-in-oil emulsion cosmetic of the present invention can further contain a microparticulate metal oxide (F) and thereby can further enhance the UV protection effect.

The metal oxide is one that is used in ordinary cosmetics, and examples thereof include titanium oxide, zinc oxide, iron oxide, cerium oxide, and chromium oxide.

The shape of the microparticulate metal oxide powder is not particularly limited and may be, for example, a spherical, flaky, rodlike, spindle-like, needle-like, or irregular shape.

The average primary particle diameter of the microparticulate metal oxide is, from the viewpoint of improving the UV protection effect, preferably from 0.005 to 0.2 $\mu$m and more preferably from 0.01 to 0.1 $\mu$m. The average primary particle diameter is the value specified in the catalogue or is measured by a transmission electron microscope.

These microparticulate metal oxides can be directly used or can also be used after hydrophobization treatment.

The hydrophobization treatment is not limited as long as it is treatment that is applied to powder for ordinary cosmetics, and a dry processing or a wet processing may be performed using a surface preparation agent, such as a fluorine compound, a silicone-based compound, metallic soap, an amino acid-based compound, lecithin, alkylsilane, an oil, or organic titanate.

Examples of the surface preparation agent include fluorine-based compounds, such as perfluoropolyether, perfluoroalkyl phosphate ester, perfluoroalkyl alkoxysilane, and fluorine-modified silicone; silicone-based compounds, such as dimethylpolysiloxane, methylhydrogenpolysiloxane, cyclic silicone, one-terminal or both-terminal trialkoxy group-modified organopolysiloxane, crosslinked silicone, silicone resin, fluorine-modified silicone resin, and acrylic-modified silicone; metallic soap, such as aluminum stearate, aluminum myristate, zinc stearate, and magnesium stearate; amino acid-based compounds, such as proline, hydroxyproline, alanine, glycine, sarcosine, glutamic acid, aspartic acid, lysine, derivatives thereof, and acylated amino acids; lecithin and hydrogenated lecithin; alkylsilanes, such as methyltrimethoxysilane, ethyltrimethoxysilane, hexyltrimethoxysilane, octyltrimethoxysilane, octyltriethoxysilane, and triethoxycaprylylsilane; oils, such as polyisobutylene, waxes, fats/oils, and fatty acid; and organic titanates, such as isopropyl triisostearoyl titanate.

The hydrophobization treatment can be performed by a usual method.

The throughput of the hydrophobization treatment is preferably from 0.1 to 15 mass%, more preferably from 1 to 12 mass%, based on the amount of the coated powder from the viewpoints of improving the dispersibility in a solvent.

The component (F) is preferably zinc oxide or titanium oxide from the viewpoints of makeup lasting quality and improving the UV protection effect.

As the component (F), the metal oxides can be used singly or in combinations of two or more, and the content thereof in the entire composition is, from the viewpoint of further enhancing the UV protection effect, preferably 0.1 mass% or more, more preferably 0.2 mass% or more, and further preferably 0.5 mass% or more and preferably 40 mass% or less, more preferably 35 mass% or less, and further preferably 30 mass% or less. In addition, the content of the component (F) in the entire composition is preferably from 0.1 to 40 mass%, more preferably from 0.2 to 35 mass%, and further preferably from 0.5 to 30 mass%.

The water-in-oil emulsion cosmetic of the present invention can further contain a color pigment (G) from the viewpoint of blurring skin imperfections, such as spots, texture of the skin, and pores, to make them less noticeable.

The color pigment is one that is used in ordinary cosmetics, and examples thereof include metal oxides, such as titanium oxide, zinc oxide, cerium oxide, aluminum oxide, yellow iron oxide, black iron oxide, colcothar, iron blue, ultramarine, chromium oxide, and chromium hydroxide; metal complexes, such as manganese violet and cobalt titanate; inorganic pigments, such as carbon black; synthetic organic pigments, organic dyes, and lake pigments thereof, such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and natural organic dyes, such as $\beta$-carotene, caramel, and paprika pigment, and include complexes of these color pigments and pigment mixtures prepared by diluting these color pigments with extender pigments, such as barium sulfate and talc.

Incidentally, as the metal oxide that is used in the component (G), those corresponding to the component (F) are excluded.

These color pigments can be directly used or can be used after hydrophobization treatment as in the component (F).

As the component (G), the color pigments can be used singly or in combinations of two or more, and the content thereof in the entire composition is, from the viewpoint of blurring skin imperfections, such as spots, texture of the skin, and pores, to make them less noticeable, preferably 0.01 mass% or more, more preferably 0.02 mass% or more, and further preferably 0.05 mass% or more and preferably 35 mass% or less, more preferably 30 mass% or less, and further preferably 25 mass% or less. In addition, the content of the component (G) in the entire composition is preferably from 0.01 to 35 mass%, more preferably from 0.02 to 30 mass%, and further preferably from 0.05 to 25 mass%.

As the component (G), the color pigments can be used singly or in combinations of two or more, and the content thereof in the entire composition is, from the viewpoints of blurring skin imperfections, such as spots, texture of the skin, and pores, to make them less noticeable and finishing the skin polished-like smooth after application, preferably 0.01 mass% or more, more preferably 0.02 mass% or more, further preferably 0.05 mass% or more, even more preferably 0.1 mass% or more, and particularly preferably 2 mass% or more and preferably 35 mass% or less, more preferably 30 mass% or less, further preferably 25 mass% or less, even more preferably 21 mass% or less, and particularly preferably 5 mass% or less. In addition, the content of the component (G) in the entire composition is preferably from 0.01 to 35 mass%, more preferably from 0.02 to 30 mass%, further preferably from 0.05 to 25 mass%, even more preferably from 0.1 to 21 mass%, and particularly preferably from 2 to 5 mass%.

The water-in-oil emulsion cosmetic of the present invention can obtain a high UV protection effect by incorporating the components (A) to (D) therein and can also obtain an effect of blurring skin imperfections, such as texture of the skin and pores, to make them less noticeable by further incorporating the color pigment (G) therein. It is preferable to incorporate the component (G) in the composition from the viewpoint of blurring skin imperfections, such as spots, texture of the skin, and pores, to make them less noticeable, but the contents of the component (A) and the component (D) are decreased by the content of the component (G), which makes difficult to balance with the component (B) in some cases. However, in the present invention, in such a case, for example, even if the total content of the components (A), (C), and (D) is 50 mass% or less, further 35 mass% or less, it is preferable to contain the component (G), because the effects of the present invention can be sufficiently exhibited while maintaining good balance of each component, and further an effect of making skin imperfections less noticeable can also be realized.

The water-in-oil emulsion cosmetic of the present invention can be a makeup cosmetic by containing the components (F) and (G), and it is possible to obtain a makeup cosmetic having a high UV protection effect, good feeling of use, and an excellent makeup effect. In this case, in particular, the cosmetic is suitable as a makeup base, a foundation, a concealer, etc. In addition, the hydrophobization treatment is preferably applied to the components (F) and (G) from the viewpoints of enhancing the adhesion to the skin and improving the makeup persistence.

The water-in-oil emulsion cosmetic of the present invention can further contain an oily gelling agent and can obtain a higher UV protection effect.

The oily gelling agent is one that is used in ordinary cosmetics and may be any oily gelling agent that can thicken the oil phase.

Examples of the oily gelling agent include an organic modified clay mineral, a crosslinked organopolysiloxane, a dextrin fatty acid ester, metallic soap, a sucrose fatty acid ester, and an oily component (wax) solid at 25°C.

The organic modified clay mineral is not limited as long as it is one that is used in ordinary cosmetics. For example, a cation-modified clay mineral obtained by treating a layered clay mineral, such as bentonite, laponite, hectorite, montmorillonite, and aluminum magnesium silicate, with a quaternary ammonium salt cationic surfactant is preferable.

Here, the quaternary ammonium salt cationic surfactant is represented by the following formula (1):

$$(R^1R^2R^3R^4N)^+ X^- \qquad (1)$$

wherein $R^1$ represents an alkyl group having 10 to 22 carbon atoms or a benzyl group; $R^2$ represents a methyl group or an alkyl group having 10 to 22 carbon atoms; $R^3$ and $R^4$ represent an alkyl group or hydroxyalkyl group having 1 to 3 carbon atoms; and X represents a halogen atom or a methylsulfate residue.

Specifically, examples thereof include dodecyltrimethylammonium chloride, myristyltrimethylammonium chloride, cetyltrimethylammonium chloride, stearyltrimethylammonium chloride, behenyltrimethylammonium chloride, myristyldimethylethylammonium chloride, cetyldimethylethylammonium chloride, stearyldimethylethylammonium chloride, behenyldimethylethylammonium chloride, myristyldiethylmethylammonium chloride, cetyldiethylmethylammonium chloride, stearyldiethylmethylammonium chloride, behenyldiethylmethylammonium chloride, benzyldimethylmyristylammonium chloride, benzyldimethylcetylammonium chloride, benzyldimethylstearylammonium chloride, benzyldimethylbehenylammonium chloride, benzylmethylethylcetylammonium chloride, benzylmethylethylstearylammonium chloride, distearyldimethylammonium chloride, and dibehenyldihydroxyethylammonium chloride; bromide compounds instead of the chlorides of the above compounds; and also dipalmitylpropylethylammonium methylsulfate.

In particular, benzyldimethylstearylammonium chloride and dimethyldistearylammonium chloride are preferable, and dimethyldistearylammonium chloride is more preferable.

The cation-modified clay mineral obtained by treating a layered clay mineral with a quaternary ammonium salt cationic surfactant is preferably dimethyldistearylammonium hectorite, dimethyldistearylammonium bentonite, or benzyldimethylstearylammonium hectorite and more preferably dimethyldistearylammonium hectorite. Examples of the commercial product thereof include Bentone 38, Bentone 38VCG, and Bentone 27 (they are manufactured by Elementis Japan K.K.).

The organic modified clay mineral can also be used as a dispersion liquid diluted with a solvent from the viewpoints of improving the workability and the excellent thickening effect of oil.

Specifically, it is preferable to use a premix gel prepared by dispersing the organic modified clay mineral in a solvent in advance. Although the solvent is not limited as long as it can be thickened by the organic modified clay mineral, octyldodecanol, a mineral oil, a volatile silicone oil, and the like are preferable from the viewpoint of the thickening effect of oil.

The content of the organic modified clay mineral in the premix gel is preferably from 5 to 25 mass%, more preferably from 10 to 20 mass%, and further preferably from 10 to 18 mass% from the viewpoints of improving the workability, the thickening effect of oil, and suppressing oil separation of the thickened oily gel itself.

As the premix gel, commercial products, such as Bentone gel EUGV and Bentone gel MIOV each containing 10 mass% of a cation-modified clay mineral, Bentone gel VS-5 PCV containing 18 mass% of a cation-modified clay mineral, and Bentone gel PTM containing 15 mass% of a cation-modified clay mineral (they are manufactured by Elementis Japan K.K.) can be used.

Examples of the crosslinked organopolysiloxane include a crosslinked dimethylpolysiloxane and a crosslinked alkylpolysiloxane, and a crosslinked polyether-modified silicone can also be used.

The crosslinked dimethylpolysiloxane is a polymer having a crosslinking structure in which a siloxane skeleton is three-dimensionally crosslinked, and the crosslinked alkylpolysiloxane further has an alkyl group having 6 to 20 carbon atoms.

Among these crosslinked organopolysiloxanes, a (dimethicone/vinyldimethicone) crosspolymer, a (dimethicone/phenyldimethicone) crosspolymer, a (vinyldimethicone/lauryldimethicone) crosspolymer, an alkyl(C30-45)cetearyl dimethicone crosspolymer, and a cetearyl dimethicone crosspolymer are preferable.

These crosslinked organopolysiloxanes may be directly used in a solid state or may be used after uniformly mixed with a liquid oil. In particular, from the viewpoints of dispersibility in a cosmetic and workability, it is preferable to be used as a dispersion liquid diluted with a liquid oil, and it is preferable to use a silicone oil, a hydrocarbon oil, or an ester oil, more preferably a silicone oil or a hydrocarbon oil, and further preferably dimethylpolysiloxane, decamethylcyclopentasiloxane, liquid paraffin, or light isoparaffin.

As the (dimethicone/vinyldimethicone) crosspolymer, commercial products, such as KSG-15 which is a mixture with decamethylcyclopentasiloxane and KSG-16 which is a mixture with low-viscosity dimethylpolysiloxane (they are manufactured by Shin-Etsu Chemical Co., Ltd.), can be used.

As the (dimethicone/phenyldimethicone) crosspolymer, commercial products, such as KSG-18 which is a mixture with methylphenylpolysiloxane (manufactured by Shin-Etsu Chemical Co., Ltd.), can be used.

As the (vinyldimethicone/lauryldimethicone) crosspolymer, commercial products, such as KSG-41 which is a mixture with liquid paraffin, KSG-42 which is a mixture with light isoparaffin, KSG-43 which is a mixture with glyceryl tri-2-ethylhexanoate, and KSG-44 which is a mixture with squalane (they are manufactured by Shin-Etsu Chemical Co., Ltd.), can be used.

As the alkyl(C30-45)cetearyl dimethicone crosspolymer, commercial products, such as Velvesil 125 which is a mixture with cyclopentasiloxane (manufactured by Momentive Performance Materials Inc.), can be used.

As the cetearyl dimethicone crosspolymer, commercial products, such as Velvesil DM which is a mixture with dimethicone (manufactured by Momentive Performance Materials Inc.), can be used.

Furthermore, as the crosslinked polyether-modified silicone, preferred are a (dimethicone/(PEG-10/15)) crosspolymer, a (PEG-15/lauryldimethicone) crosspolymer, and a (PEG-10/lauryldimethicone) crosspolymer.

The crosslinked polyether-modified silicone is preferably used as a dispersion liquid diluted with a liquid oil from the viewpoints of dispersibility in a cosmetic and workability, and a silicone oil, a hydrocarbon oil, or an ester oil is more preferably used, and a silicone oil or a hydrocarbon oil is even more preferably used.

Furthermore, the crosslinked polyether-modified silicone is preferably diluted or dispersed in a volatile hydrocarbon oil. As the volatile hydrocarbon oil, isododecane is more preferable.

As the crosslinked polyether-modified silicone, for example, commercial products, such as KSG-210 and KSG-240 (dimethicone/(PEG-10/15)) crosspolymers); KSG-310, KSG-320, and KSG-330 ((PEG-15/lauryldimethicone) crosspolymers); and KSG-340 ((PEG-10/lauryldimethicone) crosspolymer and (PEG-15/lauryldimethicone) crosspolymer) (they are manufactured by Shin-Etsu Chemical Co., Ltd.), can be used.

The dextrin fatty acid ester is an ester of fatty acid and dextrin. The number of carbon atoms of the fatty acid is preferably from 8 to 24 and more preferably from 14 to 18, the fatty acid is linear or branched saturated or unsaturated fatty acid, and the average degree of polymerization is preferably from 10 to 50 and more preferably from 20 to 30.

Specifically, examples of the dextrin fatty acid ester include dextrin palmitate, dextrin palmitate/2-ethylhexanoate, dextrin stearate, dextrin palmitate/stearate, dextrin oleate, dextrin isopalmitate, dextrin isostearate, and dextrin myristate.

Examples of the commercial product thereof include dextrin palmitate (Rheopearl KL2, Rheopearl KS2, and Rheopearl TL2), dextrin palmitate/2-ethylhexanoate (Rheopearl TT2), and dextrin myristate (Rheopearl MKL2) manufactured by Chiba Flour Milling Co., Ltd.

Examples of the oily component solid at 25°C include waxes, such as carnauba wax (melting point: 80°C to 86°C), beeswax (melting point: 64°C), candelilla wax (melting point: 68°C to 72°C), rice bran wax (melting point: 70°C to 83°C), jojoba wax (melting point: 55°C), Japan wax (melting point: 55°C), jojoba fat (melting point: 46°C to 54°C), and lanolin (melting point: 37°C to 43°C); ester oils, such as hydrogenated jojoba oil (melting point: 68°C), hydrogenated castor oil isostearate (melting point: 45°C), hydrogenated castor oil (melting point: 84°C), hydrogenated palm oil (melting point: 65°C), hardened coconut oil (melting point: 70°C), cetyl palmitate (melting point: 50°C), glyceryl behenate eicosadioate (melting point: 66°C), glyceryl behenate, tristearin, tribehenin, shea butter (melting point: 36°C to 45°C), phytosteryl/isostearyl/cetyl/stearyl/behenyl dimer dilinoleate (melting point: 38°C), glyceryl tri(caprylate/caprate/myristate/stearate) (melting point: 40°C), phytosteryl oleate, and macadamia nut fatty acid phytosteryl; ether oils, such as batyl alcohol (melting point: 60°C to 70°C) and chimyl alcohol (melting point: 60.5°C to 61.5°C); and higher alcohols, such as stearyl alcohol (melting point: 58.0°C) and behenyl alcohol (melting point: 70.5°C).

As the oily gelling agent, from the viewpoints of giving an appropriate viscosity and a uniform finish to the coating film and providing an even higher UV protection effect, preferred are an organic modified clay mineral, a crosslinked organopolysiloxane, a dextrin fatty acid ester, and an oily component solid at 25°C, and from the viewpoints of further improving the dispersion stability and improving the storage stability, more preferred are an organic modified clay mineral and an oily component solid at 25°, and further preferred are distearyldimonium hectorite and tribehenin.

As the oily gelling agent, from the viewpoints of giving an appropriate viscosity and a uniform finish to the coating film, providing an even higher UV protection effect, finishing the skin polished-like smooth after application, and reducing stickiness, it is preferable to include one or more selected from the group consisting of an organic modified clay mineral, a crosslinked organopolysiloxane, a dextrin fatty acid ester, and an oily component solid at 25°C, more preferably one or more selected from the group consisting of an organic modified clay mineral, a crosslinked organopolysiloxane, and an oily component solid at 25°C, further preferably one or more selected from the group consisting of an organic modified clay mineral and a crosslinked organopolysiloxane. In addition, it is preferable to use a combination of an organic modified clay mineral and a crosslinked organopolysiloxane.

One or more oily gelling agents can be used, and the content thereof in the entire composition is, from the viewpoint of obtaining a higher UV protection effect, preferably 0.05 mass% or more, more preferably 0.1 mass% or more, and preferably 5 mass% or less, more preferably 3 mass% or less, and further preferably 2 mass% or less. In addition, the content of the oily gelling agent in the entire composition is preferably from 0.05 to 5 mass%, more preferably from 0.1 to 3 mass%, and further preferably from 0.1 to 2 mass%.

One or more oily gelling agents can be used, and the content thereof in the entire composition is, from the viewpoints of obtaining a higher UV protection effect, finishing the skin polished-like smooth after application, and reducing stickiness, preferably 0.05 mass% or more, more preferably 0.1 mass% or more, and even more preferably 0.5 mass% or more and preferably 5 mass% or less, more preferably 3 mass% or less, further preferably 2 mass% or less, and even more preferably 1.7 mass% or less. In addition, the content of the oily gelling agent in the entire composition is preferably from 0.05 to 5 mass%, more preferably from 0.1 to 3 mass%, further preferably from 0.1 to 2 mass%, and even more preferably from 0.5 to 1.7 mass%.

In the present invention, the content of water in the entire composition is preferably from 0.1 to 50 mass%, more preferably from 1 to 30 mass%, and further preferably from 5 to 20 mass%.

The cosmetic of the present invention can contain, in addition to the above-mentioned components, components that are used in ordinary cosmetics, for example, an oil component other than the above, a powder other than the above, a surfactant other than the above, a water-soluble polymer, an antioxidant, a fragrance, a pigment, a preservative, a thickener, a pH adjuster, a blood circulation promoter, a cold sense agent, an antiperspirant, a bactericide, a skin activator, a moisturizer, and a refresher.

The water-in-oil emulsion cosmetic of the present invention can be manufactured according to a usual method and can be in a dosage form such as liquid, emulsion, paste, cream, or gel.

The water-in-oil emulsion cosmetic of the present invention can be applied as a makeup base, a foundation, and a concealer; makeup cosmetics, such as a blusher, an eyeshadow, a mascara, an eyeliner, an eyebrow, an overcoat agent, and a lipstick; UV protection cosmetics, such as a sunscreen emulsion and sunscreen cream; and skin care cosmetics, such as skin care emulsion, skin care cream, BB cream, and essence. In particular, UV protection cosmetics and makeup cosmetics are preferable. In addition, it is suitable as a makeup cosmetic containing the components (F) and (G).

With respect to the above-described embodiments, the present invention further discloses the following compositions:

<1> A water-in-oil emulsion cosmetic comprising the following components (A), (B), (C), and (D):

(A) a silicone oil (excluding component (C));
(B) a solid organic UV absorber;
(C) from 0.2 to 20 mass% of a silicone oil to which an alkyl group having 8 to 28 carbon atoms is added; and
(D) an ester oil, wherein

a mass ratio of the component (B) to the component (C), (B)/(C), is from 0.02 to 15.
<2> The water-in-oil emulsion cosmetic according to the above <1>, wherein the component (A) preferably comprises one or more selected from the group consisting of a linear dimethylpolysiloxane, a branched siloxane, and a cyclic dimethyl siloxane, more preferably comprises one or more selected from the group consisting of a linear dimethylpolysiloxane and a cyclic dimethyl siloxane, and further preferably comprises a cyclic dimethyl siloxane.

<3> The water-in-oil emulsion cosmetic according to the above <1> or <2>, wherein the component (A) is preferably a volatile silicone and more preferably comprises one or more selected from the group consisting of decamethylcyclopentasiloxane, dimethylpolysiloxane (1 cs), dimethylpolysiloxane (1.5 cs), dimethylpolysiloxane (2 cs), and methyl trimethicone, further preferably comprises one or more selected from the group consisting of decamethylcyclopentasiloxane, dimethylpolysiloxane (2 cs), and methyl trimethicone, and even more preferably at least comprises decamethylcyclopentasiloxane.

<4> The water-in-oil emulsion cosmetic according to any one of the above <1> to <3>, wherein the content of the component (A) in the entire composition is preferably 10 mass% or more, more preferably 15 mass% or more, further preferably 20 mass% or more, and even more preferably 27 mass% or more and is preferably 55 mass% or less, more preferably 50 mass% or less, further preferably 45 mass% or less, and even more preferably 34 mass% or less.

<5> The water-in-oil emulsion cosmetic according to any one of the above <1> to <4>, wherein the component (B) preferably comprises one of more selected from the group consisting of hexyl diethylaminohydroxybenzoylbenzoate, bis-ethylhexyloxyphenol methoxyphenyl triazine, and ethylhexyl triazone, more preferably one or more selected from the group consisting of hexyl diethylaminohydroxybenzoylbenzoate and bis-ethylhexyloxyphenol methoxyphenyl triazine, and preferably a combination of hexyl diethylaminohydroxybenzoylbenzoate and bis-ethylhexyloxyphenol methoxyphenyl triazine.

<6> The water-in-oil emulsion cosmetic according to any one of the above <1> to <5>, wherein the content of the component (B) in the entire composition is preferably 0.1 mass% or more, more preferably 0.2 mass% or more, further preferably 0.5 mass% or more, and even more preferably 1 mass% or more and is preferably 10 mass% or less, more preferably 7 mass% or less, further preferably 5 mass% or less, even more preferably 3 mass% or less, and particularly preferably 1.5 mass% or less.

<7> The water-in-oil emulsion cosmetic according to any one of the above <1> to <6>, wherein the component (C) is preferably caprylyl methicone, cetyl dimethicone, alkyl(C8-28)dimethicone, stearyl dimethicone, or behenoxy dimethicone and more preferably caprylyl methicone.

<8> The water-in-oil emulsion cosmetic according to any one of the above <1> to <7>, wherein the content of the component (C) in the entire composition is preferably 0.2 mass% or more, preferably 0.3 mass% or more, more preferably 0.5 mass% or more, and even more preferably 1.5 mass% or more and is 20 mass% or less, preferably 15 mass% or less, more preferably 10 mass% or less, and even more preferably 5 mass% or less.

<9> The water-in-oil emulsion cosmetic according to any one of the above <1> to <8>, wherein the mass ratio of the component (B) to the component (C), (B)/(C), is preferably 0.05 or more, more preferably 0.1 or more, and even more preferably 0.2 or more and is preferably 5 or less, more preferably 4 or less, further preferably 3 or less, and even more preferably 1 or less.

<10> The water-in-oil emulsion cosmetic according to any one of the above <1> to <9>, wherein a mass ratio of the component (C) to the component (A), (C)/(A), is preferably 0.005 or more, more preferably 0.01 or more, further preferably 0.015 or more, and even more preferably 0.045 or more and is preferably 0.5 or less, more preferably 0.4 or less, further preferably 0.3 or less, and even more preferably 0.2 or less.

<11> The water-in-oil emulsion cosmetic according to any one of the above <1> to <10>, wherein the component (D) preferably comprises one or more selected from the group consisting of 2-ethylhexyl paramethoxycinnamate, isononyl isononanoate, neopentyl glycol dicaprate, and diisostearyl malate, preferably comprises one or more selected from the group consisting of 2-ethylhexyl paramethoxycinnamate and diisostearyl malate, and preferably at least comprises 2-ethylhexyl paramethoxycinnamate.

<12> The water-in-oil emulsion cosmetic according to any one of the above <1> to <11>, wherein the content of the component (D) in the entire composition is preferably 1 mass% or more, more preferably 2 mass% or more, and further preferably 5 mass% or more and is preferably 20 mass% or less, more preferably 18 mass% or less, further preferably 15 mass% or less, and even more preferably 8 mass% or less.

<13> The water-in-oil emulsion cosmetic according to any one of the above <1> to <12>, wherein a mass ratio of the component (D) to the component (C), (D)/(C), is preferably 0.5 or more, more preferably 0.7 or more, and further preferably 1 or more and is preferably 50 or less, more preferably 30 or less, further preferably 20 or less, and even more preferably 5 or less.

<14> The water-in-oil emulsion cosmetic according to any one of the above <1> to <13>, wherein the content of the silicone-type surfactant (E) is preferably less than 5 mass%, more preferably 4.5 mass% or less, further preferably 4.0 mass% or less, even more preferably 2 mass% or less, and particularly preferably 0.5 mass% or less and is preferably 0.1 mass% or more, more preferably 0.2 mass% or more, and further preferably 0.3 mass% or more.

<15> The water-in-oil emulsion cosmetic according to the above <14>, wherein the silicone-type surfactant (E) is preferably a polyether-modified silicone surfactant, more preferably a dimethylpolysiloxane having a polyoxyethylene group or a polyoxypropylene group, and further preferably a dimethylpolysiloxane at least having a polyoxyethylene group.

<16> The water-in-oil emulsion cosmetic according to the above <14> or <15>, wherein the silicone-type surfactant (E) preferably has an HLB of 1 or more, more preferably 3 or more and preferably 12 or less, more preferably 10 or less, further preferably 8 or less, and even more preferably 6 or less.

<17> The water-in-oil emulsion cosmetic according to any one of the above <1> to <16>, preferably further comprising a microparticulate metal oxide (F), more preferably zinc oxide or titanium oxide.

<18> The water-in-oil emulsion cosmetic according to the above <17>, wherein the component (F) is preferably subjected to the hydrophobization treatment.

<19> The water-in-oil emulsion cosmetic according to the above <17> or <18>, wherein the content of the component (F) in the entire composition is preferably 0.1 mass% or more, more preferably 0.2 mass% or more, and further preferably 0.5 mass% or more and is preferably 40 mass% or less, more preferably 35 mass% or less, and further preferably 30 mass% or less.

<20> The water-in-oil emulsion cosmetic according to any one of the above <1> to <19>, preferably further comprising a color pigment (G).

<21> The water-in-oil emulsion cosmetic according to the above <20>, wherein the component (G) is preferably subjected to the hydrophobization treatment.

<22> The water-in-oil emulsion cosmetic according to the above <20> or <21>, wherein the content of the component (G) in the entire composition is preferably 0.01 mass% or more, more preferably 0.02 mass% or more, further preferably 0.05 mass% or more, even more preferably 0.1 mass% or more, and particularly preferably 2 mass% or more and is preferably 35 mass% or less, more preferably 30 mass% or less, further preferably 25 mass% or less, even more preferably 21 mass% or less, and particularly preferably 5 mass% or less.

<23> The water-in-oil emulsion cosmetic according to any one of the above <1> to <22>, comprising the microparticulate metal oxide (F) and the color pigment (G) .

<24> The water-in-oil emulsion cosmetic according to the above <23>, wherein the component (F) and the component (G) are subjected to the hydrophobization treatment.

<25> The water-in-oil emulsion cosmetic according to any one of the above <1> to <24>, preferably further comprising an oily gelling agent.

<26> The water-in-oil emulsion cosmetic according to the above <25>, wherein the oily gelling agent is preferably an organic modified clay mineral, a crosslinked organopolysiloxane, a dextrin fatty acid ester, metallic soap, a sucrose fatty acid ester, or an oily component solid at 25°C (wax), more preferably comprises one or more selected from the group consisting of an organic modified clay mineral, a crosslinked organopolysiloxane, a dextrin fatty acid ester, an oily component solid at 25°C, further preferably comprises one or more selected from the group consisting of an organic modified clay mineral, a crosslinked organopolysiloxane, and an oily component solid at 25°C, and even more preferably comprises one or more selected from the group consisting of an organic modified clay mineral and a crosslinked organopolysiloxane, and a combination of an organic modified clay mineral and a crosslinked organopolysiloxane is preferably used.

<27> The water-in-oil emulsion cosmetic according to the above <25> or <26>, wherein the content of the oily gelling agent in the entire composition is preferably 0.05 mass% or more, more preferably 0.1 mass% or more, and even more preferably 0.5 mass% or more and is preferably 5 mass% or less, more preferably 3 mass% or less, further preferably 2 mass% or less, and even more preferably 1.7 mass% or less.

<28> The water-in-oil emulsion cosmetic according to any one of the above <1> to <27>, wherein the content of water in the entire composition is preferably from 0.1 to 50 mass%, more preferably from 1 to 30 mass%, and further preferably from 5 to 20 mass%.

<29> A water-in-oil emulsion cosmetic comprising the following components (A), (B), (C), (D), and (E):

   (A) at least one silicone oil selected from the group consisting of a linear dimethylpolysiloxane, a branched siloxane, and a cyclic dimethyl siloxane (excluding component (C));
   (B) a solid organic UV absorber;
   (C) from 0.2 to 20 mass% of a silicone oil to which an alkyl group having 8 to 28 carbon atoms is added;
   (D) from 1 to 20 mass% of an ester oil; and
   (E) less than 5 mass% of a polyether-modified silicone surfactant, and

      from 0.05 to 3 mass% of an oily gelling agent, wherein
      a mass ratio of the component (B) to the component (C), (B)/(C), is from 0.02 to 15.

<30> A water-in-oil emulsion cosmetic comprising the following components (A), (B), (C), (D), and (E):

(A) at least one silicone oil selected from the group consisting of a linear dimethylpolysiloxane, a branched siloxane, and a cyclic dimethyl siloxane (excluding component (C));
(B) a solid organic UV absorber;
(C) from 0.2 to 20 mass% of a silicone oil to which an alkyl group having 8 to 28 carbon atoms is added;
(D) from 1 to 20 mass% of an ester oil; and
(E) less than 5 mass% of a polyether-modified silicone surfactant, and

from 0.05 to 2 mass% of an oily gelling agent, wherein
a mass ratio of the component (B) to the component (C), (B)/(C), is from 0.02 to 15.

<31> A water-in-oil emulsion cosmetic comprising the following components (A), (B), (C), (D), and (E):

(A) from 15 to 50 mass% of at least one silicone oil selected from the group consisting of a linear dimethylpolysiloxane, a branched siloxane, and a cyclic dimethyl siloxane (excluding component (C));
(B) a solid organic UV absorber;
(C) from 0.2 to 20 mass% of a silicone oil to which an alkyl group having 8 to 28 carbon atoms is added;
(D) from 1 to 20 mass% of an ester oil; and
(E) less than 5 mass% of a polyether-modified silicone surfactant, and

from 0.05 to 2 mass% of an oily gelling agent, wherein
a mass ratio of the component (B) to the component (C), (B)/(C), is from 0.02 to 15.

<32> A water-in-oil emulsion cosmetic comprising the following components (A), (B), (C), (D), and (E):

(A) from 15 to 50 mass% of at least one silicone oil selected from the group consisting of a linear dimethylpolysiloxane, a branched siloxane, and a cyclic dimethyl siloxane (excluding component (C));
(B) a solid organic UV absorber;
(C) from 0.2 to 20 mass% of a silicone oil to which an alkyl group having 8 to 28 carbon atoms is added;
(D) from 1 to 20 mass% of an ester oil; and
(E) 0.1 mass% or more and less than 5 mass% of a polyether-modified silicone surfactant, and

from 0.05 to 2 mass% of an oily gelling agent, wherein
a mass ratio of the component (B) to the component (C), (B)/(C), is from 0.02 to 15.

<33> A water-in-oil emulsion cosmetic comprising the following components (A), (B), (C), (D), and (E):

(A) from 15 to 50 mass% of at least one silicone oil selected from the group consisting of a linear dimethylpolysiloxane, a branched siloxane, and a cyclic dimethyl siloxane (excluding component (C));
(B) a solid organic UV absorber comprising at least one selected from the group consisting of hexyl diethylaminohydroxybenzoylbenzoate, bis-ethylhexyloxyphenol methoxyphenyl triazine, and ethylhexyl triazone;
(C) from 0.2 to 20 mass% of a silicone oil to which an alkyl group having 8 to 28 carbon atoms is added;
(D) from 1 to 20 mass% of an ester oil; and
(E) 0.1 mass% or more and less than 5 mass% of a polyether-modified silicone surfactant, and
from 0.05 to 2 mass% of an oily gelling agent comprising at least one selected from the group consisting of an organic modified clay mineral, a crosslinked organopolysiloxane, a dextrin fatty acid ester, and an oily component solid at 25°C, wherein a mass ratio of the component (B) to the component (C), (B)/(C), is from 0.02 to 15.

Examples

Examples 1 to 18 and Comparative Examples 1 to 3

Water-in-oil emulsion cosmetics of the compositions shown in Tables 1 to 3 were manufactured in the usual manner, and the SPF values were measured, and finished makeup persistence, uniformity of finish, the degree of stickiness, and the smooth-finished skin were evaluated. The results are collectively shown in Table 1.

(Evaluation method)

(1) SPF value: Each cosmetic was uniformly applied onto a PMMA plate at 1.4 mg/cm$^2$ for 1 minute and was dried in a cool dark place for 15 minutes. After the sample drying, the transmittances (%) of the absorption spectra (wavelength: 350 nm) at the midpoint on the square PMMA plate, each vertex, and the midpoint of the side connecting each vertex, 9 points in total, were measured with an SPF analyzer (UV2000S, manufactured by U.S. Labsphere Inc.), and the average of the values at 9 points was determined. The SPF values (-) were determined from the transmittances (%).

(2) Finished makeup persistence:

Three special panelists, after face washing and skin conditioning, applied each cosmetic to the whole face, and the persistence of each makeup was visually evaluated. The criteria were divided into 5 levels, in which the case where there was no change in the finish of the makeup for 6 hours was evaluated as 5, and the case where there was a change within 2 hours was evaluated as 1. The total values of the scores of the three panelists are shown as the results.

(3) Uniformity of finish:

Three special panelists, after face washing and skin conditioning, applied each cosmetic to the whole face, and the uniformity of each finish was visually evaluated. The criteria were divided into 5 levels, in which the case where there was no uneven application depending on local areas of the face was evaluated as 5, and the case where pore drop and uneven application were likely to occur and the coating film varied was evaluated as 1. The total values of the scores of the three panelists are shown as the results.

(4) Degree of stickiness:

Three special panelists, after face washing and skin conditioning, applied each cosmetic to the whole face, and the resulting coating films were subjected to sensory evaluation for the stickiness. The criteria were divided into 5 levels, in which the case where no stickiness was felt on the finished skin was evaluated as 5, and the case where unpleasant stickiness due to oil and the like was felt on the finished skin was evaluated as 1. The total values of the scores of the three panelists are shown as the results.

(5) Smooth-finished skin:

Three special panelists, after face washing and skin conditioning, applied each cosmetic to the whole face, and sensory evaluation for the finished impression was performed. The criteria were divided into 5 levels, in which the case where smooth finish was felt was evaluated as 5, and the case where smooth finish was not felt was evaluated as 1. The total values of the scores of the three panelists are shown as the results.

Incidentally, the polished-like smooth skin means that the skin looks to be three-dimensional, soft, and roundish, not flat and looks in a state in which pores and micro-irregularities on the skin are not noticeable by applying a water-in-oil emulsion cosmetic, compared to unapplied skin.

[Table 1]

| Component (mass%) | | Example | | | | | | | | | Comparative Example | | |
|---|---|---|---|---|---|---|---|---|---|---|---|---|---|
| | | 1 | 2 | 3 | 4 | 5 | 6 | 7 | 8 | 9 | 1 | 2 | 3 |
| | 95% Ethanol | 6.00 | 6.00 | 6.00 | 6.00 | 6.00 | 6.00 | 6.00 | 6.00 | 6.00 | 6.00 | 6.00 | 6.00 |
| (D) | 2-Ethylhexyl paramethoxycinnamate | 6.00 | 6.00 | 6.00 | 6.00 | 6.00 | 6.00 | 5.00 | 6.00 | 6.00 | 6.00 | 6.00 | 9.00 |
| | Diisostearyl malate | 1.20 | 1.20 | 1.20 | 1.20 | 1.20 | 1.20 | | 1.20 | 1.20 | 1.20 | 1.20 | |
| (C) | Caprylyl methicone | 1.50 | | | | | | 5.00 | 1.50 | 1.50 | | | 0.10 |
| | Cetyl dimethicone | | 1.50 | | | | | | | | | | |
| | Alkyl(C26-C28) dimethicone | | | 1.50 | | | | | | | | | |
| | (Acrylate/stearyl acrylate/ dimethicone methacrylate) copolymer | | | | 1.50 | | | | | | | | |
| | Behenoxy dimethicone | | | | | 1.50 | | | | | | | |
| | Stearyl dimethicone | | | | | | 1.50 | | | | | | |
| (A) | Methyl trimethicone | | | | | | | | | | 3.00 | | |
| | Mineral oil | | | | | | | | | | | 5.00 | |
| (B) | Hexyl diethylaminohydroxybenzoylbenzoate *1 | 0.50 | 0.50 | 0.50 | 0.50 | 0.50 | 0.50 | 0.50 | 0.50 | 0.50 | 0.50 | 0.50 | 0.50 |
| | Bis-ethylhexyloxyphenol methoxyphenyl triazine *2 | 1.00 | 1.00 | 1.00 | 1.00 | 1.00 | 1.00 | 0.50 | 1.00 | 1.00 | 1.00 | 1.00 | |
| (A) | Decamethylcyclopentasiloxane | 10.75 | 10.75 | 10.75 | 10.75 | 10.75 | 10.75 | 10.65 | 18.25 | 18.25 | 9.25 | 7.25 | 12.05 |
| (E) | PEG-8 TRIFLUOROPROPYL DIMETHICONE COPOLYMER | 0.50 | 0.50 | 0.50 | 0.50 | 0.50 | 0.50 | 0.50 | 0.50 | 0.50 | 0.50 | 0.50 | 0.50 |
| | Sorbitan monoisostearate | 0.90 | 0.90 | 0.90 | 0.90 | 0.90 | 0.90 | 0.90 | 0.90 | 0.90 | 0.90 | 0.90 | 0.90 |

EP 4 046 623 A1

(continued)

| Component (mass%) | | Example | | | | | | | | | | Comparative Example | | |
|---|---|---|---|---|---|---|---|---|---|---|---|---|---|---|
| | | 1 | 2 | 3 | 4 | 5 | 6 | 7 | 8 | 9 | 1 | 2 | 3 |
| Gelling agent | Phenyl trimethicone dispersion gel of organic modified bentonite *3 | 4.00 | 4.00 | 4.00 | 4.00 | 4.00 | 4.00 | 4.50 | | | 4.00 | 4.00 | 4.50 |
| | Dimethicone dispersion of crosslinked methylpolysiloxane *4 | 4.00 | 4.00 | 4.00 | 4.00 | 4.00 | 4.00 | 4.00 | | | 4.00 | 4.00 | 4.00 |
| | Tribehenin *5 | | | | | | | | 0.50 | | | | |
| | Dextrin palmitate *6 | | | | | | | | | 0.50 | | | |
| | Dipropylene glycol | 3.00 | 3.00 | 3.00 | 3.00 | 3.00 | 3.00 | 3.00 | 3.00 | 3.00 | 3.00 | 3.00 | 3.00 |
| | Phenoxyethanol | 0.30 | 0.30 | 0.30 | 0.30 | 0.30 | 0.30 | 0.30 | 0.30 | 0.30 | 0.30 | 0.30 | 0.30 |
| | Purified water | 19.59 | 19.59 | 19.59 | 19.59 | 19.59 | 19.59 | 19.59 | 19.59 | 19.59 | 19.59 | 19.59 | 19.59 |
| (F) | Stearic acid-treated microparticulate titanium oxide (average primary particle diameter: 15 nm) | 4.50 | 4.50 | 4.50 | 4.50 | 4.50 | 4.50 | 4.50 | 4.50 | 4.50 | 4.50 | 4.50 | 4.50 |
| | SA-treated microparticulate titanium oxide D5 dispersion (primary particle diameter: 10 to 90 nm) (SA-UT-A40/D5(50%) Mibrid Dispersion, manufactured by Miyoshi Kasei, Inc.) *12 | 2.40 | 2.40 | 2.40 | 2.40 | 2.40 | 2.40 | | 2.40 | 2.40 | 2.40 | 2.40 | |
| | Low-viscosity silicone-coated zinc oxide (primary particle diameter: 20 to 30 nm) | 3.00 | 3.00 | 3.00 | 3.00 | 3.00 | 3.00 | 3.00 | 3.00 | 3.00 | 3.00 | 3.00 | 3.00 |

(continued)

| (G) Component (mass%) | Example 1 | 2 | 3 | 4 | 5 | 6 | 7 | 8 | 9 | Comparative Example 1 | 2 | 3 |
|---|---|---|---|---|---|---|---|---|---|---|---|---|
| Octylsilylated ultrafine particulate zinc oxide dispersion liquid (average primary particle diameter: 35 nm) (45 mass% decamethylcyclopentasiloxane slurry liquid) *13 | 25.00 | 25.00 | 25.00 | 25.00 | 25.00 | 25.00 | 25.00 | 25.00 | 25.00 | 25.00 | 25.00 | 25.00 |
| Triethoxycaprylylsilane-treated titanium oxide (average primary particle diameter: 0.4 μm) | 2.50 | 2.50 | 2.50 | 2.50 | 2.50 | 2.50 | 2.50 | 2.50 | 2.50 | 2.50 | 2.50 | 2.50 |
| Dimethicone-2Na stearoyl glutamate composite treated titanium oxide (average particle diameter: 0.4μm) *7 | | | | | | | 1.20 | | | | | 1.20 |
| Dimethicone-2Na stearoyl glutamate composite treated colcothar (average particle diameter: 0.5μm) *8 | 0.05 | 0.05 | 0.05 | 0.05 | 0.05 | 0.05 | 0.05 | 0.05 | 0.05 | 0.05 | 0.05 | 0.05 |
| Dimethicone-2Na stearoyl glutamate composite treated yellow iron oxide (average particle diameter: 0.6μm) *9 | 0.30 | 0.30 | 0.30 | 0.30 | 0.30 | 0.30 | 0.30 | 0.30 | 0.30 | 0.30 | 0.30 | 0.30 |
| Dimethicone-2Na stearoyl glutamate composite treated black iron oxide (average particle diameter: 1.2μm) *10 | 0.01 | 0.01 | 0.01 | 0.01 | 0.01 | 0.01 | 0.01 | 0.01 | 0.01 | 0.01 | 0.01 | 0.01 |
| Polymethylsilsesquioxane spherical powder *11 | 3.00 | 3.00 | 3.00 | 3.00 | 3.00 | 3.00 | 3.00 | 3.00 | 3.00 | 3.00 | 3.00 | 3.00 |
| Total | 100.00 | 100.00 | 100.00 | 100.00 | 100.00 | 100.00 | 100.00 | 100.00 | 100.00 | 100.00 | 100.00 | 100.00 |

(continued)

| Component (mass%) | | Example | | | | | | | | | Comparative Example | | |
|---|---|---|---|---|---|---|---|---|---|---|---|---|---|
| | | 1 | 2 | 3 | 4 | 5 | 6 | 7 | 8 | 9 | 1 | 2 | 3 |
| Active amount | Component (A) | 28.74 | 28.74 | 28.74 | 28.74 | 28.74 | 28.74 | 27.44 | 33.20 | 33.20 | 30.24 | 25.24 | 28.84 |
| | Component (B) | 1.50 | 1.50 | 1.50 | 1.50 | 1.50 | 1.50 | 1.00 | 1.50 | 1.50 | 1.50 | 1.50 | 0.50 |
| | Component (C) | 1.50 | 1.50 | 1.50 | 1.50 | 1.50 | 1.50 | 5.00 | 1.50 | 1.50 | 0.00 | 0.00 | 0.10 |
| | Component (D) | 7.20 | 7.20 | 7.20 | 7.20 | 7.20 | 7.20 | 5.00 | 7.20 | 7.20 | 7.20 | 7.20 | 9.00 |
| | Component (E) | 0.50 | 0.50 | 0.50 | 0.50 | 0.50 | 0.50 | 0.50 | 0.50 | 0.50 | 0.50 | 0.50 | 0.50 |
| | Component (F) | 19.95 | 19.95 | 19.95 | 19.95 | 19.95 | 19.95 | 18.75 | 19.95 | 19.95 | 19.95 | 19.95 | 18.75 |
| | Component (G) | 2.86 | 2.86 | 2.86 | 2.86 | 2.86 | 2.86 | 4.06 | 2.86 | 2.86 | 2.86 | 2.86 | 4.06 |
| | Oily gelling agent | 1.56 | 1.56 | 1.56 | 1.56 | 1.56 | 1.56 | 1.64 | 0.50 | 0.50 | 1.56 | 1.56 | 1.64 |
| B/C | | 1.00 | 1.00 | 1.00 | 1.00 | 1.00 | 1.00 | 0.20 | 1.00 | 1.00 | - | - | 5.00 |
| D/C | | 4.80 | 4.80 | 4.80 | 4.80 | 4.80 | 4.80 | 1.00 | 4.80 | 4.80 | - | - | 90.00 |
| C/A | | 0.052 | 0.052 | 0.052 | 0.052 | 0.052 | 0.052 | 0.182 | 0.045 | 0.045 | 0.000 | 0.000 | 0.003 |
| SPF VALUE | | 50 | 40 | 40 | 45 | 45 | 40 | 40 | 40 | 40 | 30 | 25 | 40 |
| Finished makeup persistence | | 14 | 13 | 13 | 13 | 12 | 12 | 12 | 12 | 14 | 11 | 5 | 9 |
| Uniformity of finish | | 15 | 14 | 14 | 14 | 14 | 14 | 15 | 15 | 14 | 5 | 11 | 9 |
| Degree of stickiness | | 15 | 14 | 15 | 11 | 12 | 12 | 12 | 15 | 11 | 12 | 8 | 9 |
| Smooth finish | | 15 | 13 | 12 | 12 | 12 | 12 | 12 | 12 | 10 | 5 | 8 | 8 |

[Table 2]

| Component (mass%) | | Example | | | | | | |
|---|---|---|---|---|---|---|---|---|
| | | 10 | 11 | 12 | 13 | 14 | 15 | 16 |
| | 95% Ethanol | 6.00 | 6.00 | 6.00 | 6.00 | 6.00 | 6.00 | 6.00 |
| D | 2-Ethylhexyl paramethoxycinnamate | 7.50 | 5.00 | 5.00 | 5.00 | 9.00 | | 5.00 |
| | Isononyl isononanoate | 1.00 | | | | 1.00 | 1.00 | |
| | Neopentyl glycol dicaprate | | 2.00 | | | | 2.00 | |
| | Diisostearyl malate | 1.20 | 4.00 | | | | 5.00 | |
| C | Caprylyl methicone | 0.50 | 3.00 | 5.00 | 5.00 | 0.50 | 0.50 | 5.00 |
| | Hexyl diethylaminohydroxybenzoylbenzoate *1 | 0.50 | 0.50 | 5.00 | | 0.50 | 2.00 | 0.50 |
| B | Bis-ethylhexyloxyphenol methoxyphenyl triazine *2 | 1.00 | 2.50 | | 3.00 | | 1.00 | 0.50 |
| | Ethylhexyl triazone | | | | | | 3.00 | |
| A | Decamethylcyclopentasiloxane | 9.95 | 4.65 | 6.65 | 8.65 | 10.65 | 7.15 | 10.65 |
| E | Fluorine polyether co-modified silicone | 0.50 | 0.50 | 0.50 | 0.50 | 0.50 | 0.50 | 0.50 |
| | Sorbitan monoisostearate | 0.90 | 0.90 | 0.90 | 0.90 | 0.90 | 0.90 | 0.90 |
| Gelling agent | Organic modified bentonite dispersing gel *3 | 4.50 | 4.50 | 4.50 | 4.50 | 4.50 | 4.50 | 4.50 |
| | Crosslinked methylpolysiloxane dispersion *4 | 4.00 | 4.00 | 4.00 | 4.00 | 4.00 | 4.00 | 4.00 |
| | Dipropylene glycol | 3.00 | 3.00 | 3.00 | 3.00 | 3.00 | 3.00 | 3.00 |
| | Phenoxyethanol | 0.30 | 0.30 | 0.30 | 0.30 | 0.30 | 0.30 | 0.30 |
| | Purified water | 19.59 | 19.59 | 19.59 | 19.59 | 19.59 | 19.59 | 19.59 |
| F | Stearic acid-treated microparticulate titanium oxide | 4.50 | 4.50 | 4.50 | 4.50 | 4.50 | 4.50 | 4.50 |
| | Low-viscosity silicone-coated zinc oxide | 3.00 | 3.00 | 3.00 | 3.00 | 3.00 | 3.00 | 3.00 |
| | Octylsilylated ultrafine particulate zinc oxide dispersion liquid (average primary particle diameter: 35 nm) (45 mass% decamethylcyclopentasiloxane slurry liquid) *13 | 25.00 | 25.00 | 25.00 | 25.00 | 25.00 | 25.00 | 25.00 |

EP 4 046 623 A1

(continued)

| Component (mass%) | | Example | | | | | | |
|---|---|---|---|---|---|---|---|---|
| | | 10 | 11 | 12 | 13 | 14 | 15 | 16 |
| G | Triethoxycaprylylsilane-treated titanium oxide | 2.50 | 2.50 | 2.50 | 2.50 | 2.50 | 2.50 | 2.50 |
| | Dimethicone-2Na stearoyl glutamate composite treated titanium oxide *7 | 1.20 | 1.20 | 1.20 | 1.20 | 1.20 | 1.20 | 1.20 |
| | Dimethicone-2Na stearoyl glutamate composite treated colcothar *8 | 0.05 | 0.05 | 0.05 | 0.05 | 0.05 | 0.05 | 0.05 |
| | Dimethicone-2Na stearoyl glutamate composite treated yellow iron oxide *9 | 0.30 | 0.30 | 0.30 | 0.30 | 0.30 | 0.30 | 0.30 |
| | Dimethicone-2Na stearoyl glutamate composite treated black iron oxide *10 | 0.01 | 0.01 | 0.01 | 0.01 | 0.01 | 0.01 | 0.01 |
| | Polymethylsilsesquioxane spherical powder | 3.00 | 3.00 | 3.00 | 3.00 | 3.00 | 3.00 | 3.00 |
| | Total | 100.00 | 100.00 | 100.00 | 100.00 | 100.00 | 100.00 | 100.00 |
| Active amount | Component (A) | 26.74 | 21.44 | 23.44 | 25.44 | 27.44 | 23.94 | 27.44 |
| | Component (B) | 1.50 | 3.00 | 5.00 | 3.00 | 0.50 | 6.00 | 1.00 |
| | Component (C) | 0.50 | 3.00 | 5.00 | 5.00 | 0.50 | 0.50 | 5.00 |
| | Component (D) | 9.70 | 11.00 | 5.00 | 5.00 | 10.00 | 8.00 | 5.00 |
| | Component (E) | 0.50 | 0.50 | 0.50 | 0.50 | 0.50 | 0.50 | 0.50 |
| | Component (F) | 18.75 | 18.75 | 18.75 | 18.75 | 18.75 | 18.75 | 18.75 |
| | Component (G) | 4.06 | 4.06 | 4.06 | 4.06 | 4.06 | 4.06 | 4.06 |
| | Oily gelling agent | 1.64 | 1.64 | 1.64 | 1.64 | 1.64 | 1.64 | 1.64 |
| | B/C | 3.00 | 1.00 | 1.00 | 0.60 | 1.00 | 12.00 | 0.20 |
| | D/C | 19.4 | 3.7 | 1 | 1 | 20 | 16 | 1 |
| | C/A | 0.019 | 0.140 | 0.213 | 0.197 | 0.018 | 0.021 | 0.182 |
| | A/(C+D) | 2.62 | 1.53 | 2.34 | 2.54 | 2.61 | 2.82 | 2.74 |
| | SPF VALUE | 60 | 45 | 40 | 40 | 50 | 50 | 40 |
| | Finished makeup persistence | 14 | 13 | 13 | 14 | 13 | 13 | 13 |
| | Uniformity of finish | 14 | 15 | 14 | 15 | 13 | 13 | 13 |
| | Degree of stickiness | 14 | 12 | 12 | 12 | 12 | 14 | 14 |
| | Smooth finish | 15 | 14 | 14 | 15 | 14 | 14 | 13 |

[Table 3]

| Component (mass%) | | | Example | |
|---|---|---|---|---|
| | | | 17 | 18 |
| | | 95% Ethanol | 6.00 | 6.00 |
| (D) | | 2-Ethylhexyl paramethoxycinnamate | 15.00 | 2.00 |
| (C) | | Caprylyl methicone | 10.00 | 0.50 |
| (B) | | Hexyl diethylaminohydroxybenzoylbenzoate *1 | 0.20 | 3.00 |
| | | Bis-ethylhexyloxyphenol methoxyphenyl triazine *2 | | 2.00 |
| | | Ethylhexyl triazone | | 2.00 |
| (A) | | Dimethylpolysiloxane (2 cs) | 14.15 | 33.50 |
| | | Methyl trimethicone | 5.00 | 5.00 |
| (E) | | PEG-10 DIMETHICONE | 2.00 | 0.50 |
| | | Sorbitan monoisostearate | 0.90 | 0.90 |
| Gelling agent | | Phenyl trimethicone dispersion gel of organic modified bentonite *3 | 1.00 | |
| | | Dimethicone dispersion of crosslinked methylpolysiloxane *4 | | 8.30 |
| | | Dipropylene glycol | 3.00 | 3.00 |
| | | Phenoxyethanol | 0.30 | 0.30 |
| | | Purified water | 12.35 | 12.16 |
| (F) | | Stearic acid-treated microparticulate titanium oxide (average primary particle diameter: 15 nm) | 30.00 | 0.50 |
| (G) | | Triethoxycaprylylsilane-treated titanium oxide (average primary particle diameter: 0.4 μm) | 0.05 | 17.65 |
| | | Dimethicone-2Na stearoyl glutamate composite treated colcothar (average particle diameter: 0.5μm) *8 | 0.02 | 0.47 |
| | | Dimethicone-2Na stearoyl glutamate composite treated yellow iron oxide (average particle diameter: 0.6μm) *9 | 0.02 | 1.98 |
| | | Dimethicone-2Na stearoyl glutamate composite treated black iron oxide (average particle diameter: 1.2μm) *10 | 0.01 | 0.24 |
| | | Total | 100.00 | 100.00 |
| Active amount | | Component (A) | 20.00 | 44.81 |
| | | Component (B) | 0.20 | 7.00 |
| | | Component (C) | 10.00 | 0.50 |
| | | Component (D) | 15.00 | 2.00 |
| | | Component (E) | 2.00 | 0.50 |
| | | Component (F) | 30.00 | 0.5 |
| | | Component (G) | 0.10 | 20.34 |
| | | Oily gelling agent | 0.15 | 1.99 |
| B/C | | | 0.02 | 14.00 |
| D/C | | | 1.50 | 4.00 |
| C/A | | | 0.500 | 0.011 |
| SPF VALUE | | | 50 | 50 |

(continued)

| Component (mass%) | Example | |
| --- | --- | --- |
| | 17 | 18 |
| Finished makeup persistence | 13 | 12 |
| Uniformity of finish | 13 | 13 |
| Degree of stickiness | 12 | 13 |
| Smooth finish | 12 | 10 |

The trade names and the like of the components used in Examples are as follows:

*1: UVINUL A PLUS GRANULAR (manufactured by BASF SE);
*2: TINOSORB S (manufactured by BASF SE);
*3: BENTONE GEL PTM (manufactured by Elementis Japan K.K.): 15 mass% of pure substance and 85 mass% of phenyltrimethicone;
*4: KSG-210 (manufactured by Shin-Etsu Chemical Co., Ltd.): 24 mass% of pure substance and 76 mass% of methyl trimethicone (methyl trimethicone in KSG-210 was included in the content of the component (A));
*5: Syncrowax HR-C (manufactured by Croda International plc) ;
*6: Rheopearl KL2 (manufactured by Chiba Flour Milling Co., Ltd.);
*7: SA/NAI-TR-10 MIBRID COLOR POWDER (manufactured by Miyoshi Kasei, Inc.);
*8: SA/NAI-R-10 MIBRID COLOR POWDER (manufactured by Miyoshi Kasei, Inc.);
*9: SA/NAI-Y-10 MIBRID COLOR POWDER (manufactured by Miyoshi Kasei, Inc.)
*10: SA/NAI-B-10 MIBRID COLOR POWDER (manufactured by Miyoshi Kasei, Inc.);
*11: Tospearl 145A (manufactured by Momentive Inc.);
*12: D5 in SA-treated microparticulate titanium oxide D5 dispersion (primary particle diameter: 10 to 90 nm) shows decamethylcyclopentasiloxane and was included in the content of the component (A); and
*13: Decamethylcyclopentasiloxane in octylsilylated ultrafine particulate zinc oxide dispersion liquid (45 mass% decamethylcyclopentasiloxane slurry liquid) was included in the content of the component (A).

Prescription Example 1

A water-in-oil emulsion cosmetic of the composition shown in Table 4 was manufactured in the usual manner.
The resulting water-in-oil emulsion cosmetic had an SPF value of 50, and the makeup persistence of the finish was excellent, the finish was uniform, there was no stickiness, and the smooth-finished skin was obtained.

[Table 4]

| | Component (mass%) | Prescri ption Exampl e |
| --- | --- | --- |
| | | 1 |
| | 95% Ethanol | 6.00 |
| (D) | Isononyl isononanoate | 1.00 |
| | Neopentyl glycol dicaprate | 2.00 |
| | Diisostearyl malate | 5.00 |
| (C) | Caprylyl methicone | 0.50 |
| (B) | Hexyl diethylaminohydroxybenzoylbenzoate *1 | 2.00 |
| | Bis-ethylhexyloxyphenol methoxyphenyl triazine *2 | 1.00 |
| | Ethylhexyl triazone | 3.00 |

(continued)

| | Component (mass%) | | Prescription Example 1 |
|---|---|---|---|
| (A) | Decamethylcyclopentasiloxane | | 7.15 |
| (E) | Fluorine polyether co-modified silicone | | 0.50 |
| | Sorbitan monoisostearate | | 0.90 |
| Gelling agent | Phenyl trimethicone dispersion gel of organic modified bentonite *3 | | 4.50 |
| | Dimethicone dispersion of crosslinked methylpolysiloxane *4 | | 4.00 |
| | Dipropylene glycol | | 3.00 |
| | Phenoxyethanol | | 0.30 |
| | Purified water | | 19.59 |
| (F) | Stearic acid-treated microparticulate titanium oxide (average primary particle diameter: 15 nm) | | 4.50 |
| | Low-viscosity silicone-coated zinc oxide (primary particle diameter: 20 to 30 nm) | | 3.00 |
| | Octylsilylated ultrafine particulate zinc oxide dispersion liquid (average primary particle diameter: 35 nm) (45 mass% decamethylcyclopentasiloxane slurry liquid) *13 | | 25.00 |
| (G) | Triethoxycaprylylsilane-treated titanium oxide (average primary particle diameter: 0.4 μm) | | 2.50 |
| | Dimethicone-2Na stearoyl glutamate composite treated titanium oxide (average particle diameter: 0.4μm) *7 | | 1.20 |
| | Dimethicone-2Na stearoyl glutamate composite treated colcothar (average particle diameter: 0.5μm) *8 | | 0.05 |
| | Dimethicone-2Na stearoyl glutamate composite treated yellow iron oxide (average particle diameter: 0.6μm) *9 | | 0.30 |
| | Dimethicone-2Na stearoyl glutamate composite treated black iron oxide (average particle diameter: 1.2μm) *10 | | 0.01 |
| | Polymethylsilsesquioxane spherical powder *11 | | 3.00 |
| | Total | | 100.00 |
| Active amount | Component (A) | | 23.94 |
| | Component (B) | | 6.00 |
| | Component (C) | | 0.50 |
| | Component (D) | | 8.00 |
| | Component (E) | | 0.50 |
| | Component (F) | | 18.75 |
| | Component (G) | | 4.06 |
| | Oily gelling agent | | 6.87 |
| | B/C | | 12.00 |
| | D/C | | 16.00 |
| | C/A | | 0.021 |

**Claims**

1. A water-in-oil emulsion cosmetic comprising following components (A), (B), (C), and (D):

    (A) a silicone oil (excluding component (C));
    (B) a solid organic UV absorber;
    (C) from 0.2 to 20 mass% of a silicone oil to which an alkyl group having 8 to 28 carbon atoms is added; and
    (D) an ester oil, wherein

    a mass ratio of the component (B) to the component (C), (B)/(C), is from 0.02 to 15.

2. The water-in-oil emulsion cosmetic according to Claim 1, wherein a mass ratio of the component (D) to the component (C), (D)/(C), is from 0.5 to 50.

3. The water-in-oil emulsion cosmetic according to Claim 1 or 2, wherein a content of a polyether-modified silicone surfactant (E) is less than 5 mass%.

4. The water-in-oil emulsion cosmetic according to any one of Claims 1 to 3, further comprising a microparticulate metal oxide (F) and a color pigment (G).

5. The water-in-oil emulsion cosmetic according to Claim 4, wherein the component (F) and the component (G) are subjected to a hydrophobization treatment.

6. The water-in-oil emulsion cosmetic according to any one of Claims 1 to 5, further comprising an oily gelling agent.

7. The water-in-oil emulsion cosmetic according to any one of Claims 1 to 6, wherein a content of the component (D) is from 1 to 20 mass%.

8. A water-in-oil emulsion cosmetic comprising following components (A), (B), (C), (D), and (E):

    (A) at least one silicone oil selected from the group consisting of a linear dimethylpolysiloxane, a branched siloxane, and a cyclic dimethyl siloxane (excluding component (C));
    (B) a solid organic UV absorber;
    (C) from 0.2 to 20 mass% of a silicone oil to which an alkyl group having 8 to 28 carbon atoms is added;
    (D) from 1 to 20 mass% of an ester oil; and
    (E) less than 5 mass% of a polyether-modified silicone surfactant, and

    from 0.05 to 3 mass% of an oily gelling agent, wherein
    a mass ratio of the component (B) to the component (C), (B)/(C), is from 0.02 to 15.

## INTERNATIONAL SEARCH REPORT

International application No.

PCT/JP2020/038661

### A. CLASSIFICATION OF SUBJECT MATTER

Int.Cl. A61K8/891(2006.01)i, A61K8/06(2006.01)i, A61Q19/00(2006.01)i
FI: A61K8/891, A61K8/06, A61Q19/00

According to International Patent Classification (IPC) or to both national classification and IPC

### B. FIELDS SEARCHED

Minimum documentation searched (classification system followed by classification symbols)
Int.Cl. A61K8/891, A61K8/06, A61Q19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2016/068298 A1 (SHISEIDO CO., LTD.) 06 May 2016 (2016-05-06), claim 1, paragraphs [0017], [0018], [0081] | 1-8 |
| X | JP 2018-70556 A (KOSE CORPORATION) 10 May 2018 (2018-05-10), claims, paragraphs [0032], [0040] | 1-8 |
| A | JP 2011-236201 A (SHISEIDO CO., LTD.) 24 November 2011 (2011-11-24), paragraph [0058] | 1-8 |

☐ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 November 2020 | 08 December 2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/038661

| | | |
|---|---|---|
| WO 2016/068298 A1 | 06 May 2016 | US 2017/0333301 A1<br>claim 1, paragraphs [0024]-[0024],<br>[0097]<br>EP 3213742 A1 |
| JP 2018-70556 A | 10 May 2018 | (Family: none) |
| JP 2011-236201 A | 24 November 2011 | US 2013/0022563 A1<br>paragraph [0086] |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015189671 A
- JP 2005232069 A
- JP 2018108952 A